(19) **Europäisches Patentamt**
**European Patent Office**
**Office européen des brevets**

(11) **EP 4 394 777 A1**

(12) **EUROPEAN PATENT APPLICATION**

(43) Date of publication:
**03.07.2024 Bulletin 2024/27**

(51) International Patent Classification (IPC):
**G16B 30/10** (2019.01)     **G16B 50/30** (2019.01)

(21) Application number: **22383313.8**

(52) Cooperative Patent Classification (CPC):
**G16B 30/10; G16B 50/30**

(22) Date of filing: **29.12.2022**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Barcelona Supercomputing Center-Centro Nacional de Supercomputación**
**08034 Barcelona (ES)**

(72) Inventors:
- **DOBLAS FONT, Max**
  **08022 Barcelona (ES)**
- **LOSTES CAZORLA, Oscar**
  **08032 Barcelona (ES)**
- **MARCO SOLA, Santiago**
  **08028 Barcelona (ES)**
- **MORETÓ PLANAS, Miquel**
  **08960 Sant Just Desvern (ES)**

(74) Representative: **TRBL Intellectual Property**
**Glorieta de Quevedo, 8**
**28015 Madrid (ES)**

(54) **METHOD FOR SEQUENCE ALIGNMENT OPTMIZATION**

(57)     The present invention discloses a method and computation module (11) for implementing said method for accelerating an alignment algorithm to align sequences (1) of information in which, from the initial algorithm, two input vectors (9, 10) based on differential encoding allocating vertical and horizontal differences of the edit distance of the sequences (1) are computed. These vectors (9, 10) are used to compute a first tile (7) of dimension T x T, both for the vertical and horizontal difference matrices (5, 6), and only the last column or row within the tile (7) is stored in memory. This computation is repeated for adjacent tiles (7) using as input the adjacent column or row stored. Finally, from a starting position, the traceback (4) of a whole tile (7) of dimension T x T is computed and the ending position of such traceback (4) is stored in memory. The aligned sequences (1) corresponding to the traceback (4) of the tile (7) are stored and the traceback (4) of a new, adjacent tile (7) is computed in the same way until the sequences (1) have been aligned.

**FIG. 4**

**Description**

**FIELD OF THE INVENTION**

The present invention belongs to the field of computer-implemented methods, and, more specifically, to a method for accelerating an alignment algorithm to establish a correspondence between two or more sequences of information, being those, mainly, genetic sequences, based on the computation of the degree of similarity between said sequences and related to the field of dynamic programming. Specific, tailored hardware implementations of said method are also disclosed.

**BACKGROUND OF THE INVENTION**

Sequence alignment aims to determine the differences between two or more sequences of information. According to a given error model, alignment distance is defined as the minimum number of operations (i.e., mismatch, insertion, and deletion) required to transform one sequence into another. Many different error models have been proposed to minimize the transformation cost. Undoubtedly, the most fundamental and well-studied error model is the so-called edit distance, as it is powerful enough for a wide range of applications.

Sequence alignment is a fundamental problem in many application domains including information retrieval, natural language processing, security, data mining, optical character recognition, spell correction, and pattern matching, among others. This later case relates tightly to the advent of genome sequencing technologies, thanks to which sequence alignment has acquired special relevance in computational biology and genome sequence analysis. Modern sequencing machines can rapidly produce millions of relatively small DNA sequences ranging from a few hundred of base pairs (bps) to millions of them (megabase pairs) at a low cost. For the past decade, genomic data production has been doubling every 7 months, notably outpacing Moore's Law, and even surpassing other big data sources such as those coming from YouTube and Twitter. If this trend continues, it will be possible soon to sequence billions of whole human genomes yearly, generating exabytes of raw genomic data.

This increase in genomic data has been crucial for the development of population-wide genetic studies, diagnosis of diseases such as cancer, autism or diabetes and personalized healthcare, effective outbreak tracing (COVID-19, Ebola), biodiversity preservation, and even DNA-based computing and storage systems. Increasing production yields and sequence lengths pose a computational challenge for current genome sequence analysis tools and their associated hardware accelerators. As a result, the performance bottleneck in genome sequence analysis is moving from the physical process of genetic sequencing to the computational post-processing and analysis. In particular, accelerating ubiquitous building blocks, like sequence alignment, have become paramount to bridging the gap between sequence data production and current computing power.

Since the completion of the first human genome, sequencing technologies have rapidly evolved to produce longer sequences, increasing the data-production throughput of their machines while reducing operational costs. Currently available sequencing technologies can be broadly classified into second- and third-generation sequencing technologies. Second-generation sequencing technologies, like Illumina and Ion Torrent, have dominated the market for the last decade. These technologies generate short sequences (i.e., 100 - 300 bps) of high quality (i.e., <1% error rate). Third-generation sequencing technologies, like Pacific Biosciences (PacBio) and Oxford Nanopore Technologies (ONT), improve upon previous generations to produce megabase-size sequences. Unfortunately, these technologies are more error-prone, producing a typical error rate of 10%-15%. Genome sequencing data requires complex and computationally intensive analyses before they can be meaningful to researchers and clinicians.

This analysis has typically relied upon sequence alignment algorithms based on dynamic programming (DP), that scale quadratically in both execution time and memory with the length of the sequences involved. As a result, tools requiring sequence alignment become the bottleneck of many genome sequence analyses and fail to scale with longer sequence lengths. Usually, classical sequence alignment is computed using some variation of DP and consists of two phases: DP-matrix computation and alignment traceback. During the first phase, sequence alignment algorithms compute a DP-matrix of $n \times m$ elements with $n$ and $m$ being the lengths of the sequences to be aligned. Each element of this matrix corresponds to a correspondence score (usually, an integer) between two characters or pieces of the sequences and depends on the left, upper-left and upper adjacent elements. Then, for the traceback phase, sequence alignment algorithms trace the list of operations that led to the optimum distance (bottom-right element of the matrix) back to the beginning (upper-left element); that is, the alignment between the sequences.

Sequence alignment algorithms and accelerators have been intensively studied for more than 60 years. Many techniques have been proposed over the years to accelerate sequence alignment computations on software and hardware. Nevertheless, classical DP-based solutions require quadratic time and memory on the sequence length. Hence, aligning large volumes of increasingly long sequences poses a challenge to the scalability of these algorithms.

Bit-parallel techniques emerged in the 80s as an effective strategy to accelerate sequence alignment algorithms. These techniques exploit bitwise operations to compute multiple elements of the DP-matrix in parallel. Most importantly, they map extremely well to conventional hardware instructions, outperforming other sequence alignment approaches in practice. As a result, these techniques have been widely adopted by many software tools and hardware accelerators over the years. In 1989, the first bit-parallel algorithm, called - Bitap, was proposed. Bitap reformulates the problem using a DP-matrix of $n \times k$ bits, where $k$ is the maximum edit distance supported, requiring $k$ bits per element of the original DP-matrix. Then, it progressively computes the edit distance, recomputing the whole bit-matrix per each character of text aligned. Thus, the alignment computations are proportional to $O(nkm)$. However, this ingenious reformulation allows for computing the elements of each column independently using bitwise operations. Assuming a sufficiently large machine word ($w$ bits), bitap's computation reduces from $O(nwkm)$ to $O(km)$ and requires $7 \cdot k$ bitwise instructions per character aligned. Bitap was designed to compute the edit distance between short sequences (i.e., that fit in a machine word) with a low alignment error, as its complexity depends on the maximum error supported, $k$. Originally, it was not designed to compute the complete alignment, as it requires storing the $m$ DP-matrices of $n \times k$ bits to perform the traceback phase. Bitap was recently resurrected as the bedrock algorithm for hardware accelerators, like GenASM and SeGraM. These domain-specific accelerators extended Bitap, using large bit-vectors and tailored bitwise logic to accelerate the computation of Bitap's $n \times k$ bit-matrix per each character aligned.

Ten years later, Myers proposed a novel bit-parallel error-agnostic algorithm that outperforms the Bitap algorithm, scaling to longer sequences and higher error rates. Broadly known as Bit-Parallel Myer's (BPM), this algorithm benefits from the observation that differences between adjacent row and column elements are limited to {-1, 0, +1}. BPM exploits this property encoding vertical differences, $\Delta v_{i,j} = H_{i,j} - H_{i-1,j}$, and horizontal differences $\Delta h_{i,j} = H_{i,j} - H_{i,j-1}$, with $H$ being the DP-matrix, requiring only ($2 \times 2$) bits per element of the original DP-matrix, irrespective of the alignment error. Then, it reformulates the classical DP methodology in terms of differences, where each variable can be encoded using 2 bits.

The BPM proposes to compute the DP-matrix column-wise, packing the elements of each column in a bit-vector. Using a cunning and convoluted strategy, the BPM manages to compute each bit-encoded column using only 17 CPU instructions per character aligned. As a result, the BPM requires to perform $O(n\,wm)$ computations (or $O(m)$ if $n < 2w$). In practice, the BPM outperforms other bit-parallel algorithms, scaling to longer sequences and higher error rates. Variations of the BPM technique have been broadly adopted by multiple tools in genome sequence analysis like Edlib and Daligner.

Nonetheless, and although many software and hardware sequence alignment accelerators have been proposed over the years, they present different limitations.

First of all, they present performance limitations. Classic DP-based sequence alignment algorithms are heavily restricted by quadratic time and memory requirements with respect to the length of the sequences to be aligned. Due to the intrinsic dependencies between computations of the DP-matrix, computational parallelism is significantly constrained. To alleviate this problem, many hardware accelerators rely on bit-parallel techniques due to their convenient mapping to bitwise operations. However, bit-parallel accelerators still suffer from computational bottlenecks and limited memory bandwidth. Accelerators based on Bitap obtain computational parallelism at the expense of increasing computations to $O(nwm)$ and require implementing large bit-vectors ($w > n$) to compensate. Moreover, larger memory requirements (i.e., $n \cdot k$ bit-matrix) put higher pressure on memory bandwidth. These performance limitations escalate with increasing error rates, as bitap's complexity is sensitive to alignment error ($k$). In contrast, BPM's complexity $O(nwm)$ is not sensitive to the alignment error. Nevertheless, accelerators based on BPM still require performing a quadratic number of operations and storing $4 \times n \times m$ bits. On top of that, bit-parallel techniques require additional pre-processing steps that put a non-negligible toll on performance. Further, the parallelism of these solutions remains limited to processing one character at a time, presenting a reduced computational intensity and large bandwidth requirements.

There also exist scalability limitations concerning these accelerators. The quadratic complexity of DP-based algorithms poses a challenge to scale with longer sequence lengths and higher error rates. For instance, computing the complete alignment of 10kbp-long sequences, assuming just an error of 0.1%, would require 381.4MB of memory for the classical DP algorithm, 119.2MB for Bitap, and 47.6MB for BPM. As the sequence length increases, bit-parallel based accelerators require larger hardware bit-vectors, increasing chip area and energy consumption. In the case of Bitap, being sensitive to the alignment error poses an additional limitation to scale to high-error rates. In an attempt to overcome these difficulties, many solutions are limited to computing the edit distance (not the complete alignment) or fall back to heuristic algorithms that compute small portions of the DP-matrix, compromising the accuracy of the results. As a result, these methods present dire scalability issues when aligning long and noisy sequences, like those ones produced by third-generation sequencing technologies.

When trying to overcome these limitations, many sequence alignment accelerators present solutions that improve performance and scalability at the cost of accuracy. Some of these solutions limit the maximum alignment error tolerated $k$, computing only some cells around the main diagonal of the DP-matrix (banded alignment). Others compute local alignments between small portions of the sequences and combine them afterwards to approximate the optimal alignment (windowed alignment). As a result, the accuracy of these heuristics is unpredictable and often leads to sub-optimal results. In turn, these inexact results jeopardize the overall accuracy of the downstream analysis tools, leading to invalid genome sequence analysis insights.

On the other hand, many of these solutions suffer from efficiency limitations. Domain-specific accelerators show significant performance improvements at the expense of complex and expensive hardware designs, both in area and energy. The efficiency limitations of these accelerators ponder whether their benefits outperform their costs. In particular, bit-parallel accelerators implementing large bit-vectors tend to consume significant chip area and energy. Moreover, monolithic hardware accelerators overspecialized in genome resequencing invest many resources to support other functionalities (e.g., indexing and seeding) not required beyond genome mapping. Furthermore, loosely-coupled accelerators require complex hardware designs to implement data coherence and transfers. Concerning the latter, these accelerators often incur in expensive data transfers that diminish the performance gains when integrated into production-ready tools.

Finally, it is to be noted that these methods present applicability limitations as well. Seeking performance improvements, domain-specific accelerators tend to focus on specific use cases and input characteristics. Overspecialization often tampers the applicability of these accelerators to related use cases. In practice, integrating these accelerators into production-ready tools can be daunting, requiring extensive modification of the software stack. Monolithic accelerators often result inflexible to be repurposed or extended for other applications.

The need for better sequence alignment algorithms has fostered extensive research on algorithms and hardware accelerators for sequence alignment including solutions based on GPUs, FPGAs, PIM, and ASICs. Notable solutions, like GenAx and GenASM, based on computing edit distance have demonstrated that domain-specific accelerators can deliver significant performance improvements. However, optimized software-hardware co-designs are often tailored to specific use cases (sequence mapping) and fail to scale to different workloads (longer sequences or higher error rates).

In terms of hardware, there exist a number of works that have explored, for instance, using processing in memory (PIM) for sequence alignment. One notable example is RAPID, which accelerates the computation of the DP-matrix by processing entire antidiagonals at once, exploiting the large amount of parallelism available in PIM architectures. As opposed, BioHD uses hyperdimensional computing to calculate the edit distance exploiting PIM to accelerate the computation. Nevertheless, while these architectures often share some portion of the memory hierarchy with the CPU, they are still not integrated into the CPU pipeline. Relevant studies like SeedEx, GenAx, Darwin, GenASM, and SeGraM. SeedEx propose custom hardware designs to accelerate the computation of a portion of the DP-matrix using a heuristic approach. While SeedEx presents an FPGA-based solution, Darwin proposes the design of an ASIC. In contrast, GenAx takes a slightly different approach, developing a hardware automation for computing the edit distance. In the case of GenASM and SeGraM, both present hardware accelerators based on the Bitap algorithm for the computation of the edit distance. All these accelerators are, however, all loosely coupled and exist outside the CPU pipeline.

A related but orthogonal field of work is the design of algorithms to improve the speed of alignment on general purpose hardware. There are works focused on algorithmic improvements for single-threaded performance, better utilization of parallel processing, taking advantage of single-instruction multiple-data (SIMD) instructions, and using GPUs to accelerate sequence alignment. These works use a variety of different algorithms, which vary in their amenability to adding specialized hardware to accelerate them. However, these algorithms, like Edlib, which implements BPM, still suffer from the problems mentioned above. Another example is Nvidia, that recently announced the DPX instruction set extension to accelerate DP-matrix computations on GPU, as presented in patent application US 20210048992 A1. Nevertheless, upcoming DPX extensions are limited to computing a single DP-element per instruction, exploiting the multiple computing units of the GPU to perform DP-matrix computations in parallel.

As exposed, software and hardware sequence alignment accelerators proposed over the years present different limitations. It is therefore of great interest, given its importance in applications such as genomic analysis, to provide a solution that can meet the performance demands of sequence analysis tools, being fast, scalable, accurate, and efficient at the same time.

## SUMMARY OF THE INVENTION

In order to mitigate the shortcomings mentioned above, the present invention proposes a method for the alignment of at least two sequences of information (generic characters, genomic data, time-series data and others) based on computing the differential encodings of the edit distances corresponding to said sequences in tiles of dimension $T \times T$, with T being an integer larger than 1. This allows to store exclusively the elements of only one column and/or row of each tile, i.e., the elements at the edges of each tile, saving memory and obtaining fastest results for the alignment of the sequences. Furthermore, the present invention also proposes a computation module configured to perform said method which is specifically designed for this purpose.

With these characteristics, advantageously, a first embodiment of the invention consists of a computer-implemented method for accelerating an alignment algorithm to establish a correspondence between at least two sequences of information comprising the following steps:

  a) receiving, in a computation module, at least two sequences of information;

b) storing said sequences in at least one memory unit of the computation module;

c) computing, with the computation module, a vertical input vector and a horizontal input vector based on differential encodings of scores associated to the comparison of the sequences according to the alignment algorithm to be accelerated;

and **characterized in that** the method further comprises the following steps:

d) setting a value corresponding to a matrix tile size, T, being said value an integer larger than 1;

e) computing, from the first T elements of the vertical input vector determined in step c), a vertical difference matrix of a first tile of dimension $T \times T$ according to the alignment algorithm, and storing in the memory unit the values of the column with the highest column index within the tile;

f) computing, from the first T elements of the horizontal input vector determined in step c), a horizontal difference matrix of a first tile of dimension $T \times T$ according to the alignment algorithm, and storing in the memory unit the values of the row with the highest row index within the tile;

g) repeating step e) iteratively for at least one adjacent non-overlapping tile in the vertical difference matrix, using as input the stored values of the column immediately before said adjacent non-overlapping tile, or using the corresponding T elements of the vertical input vector computed in step c) if there are no such values stored because said adjacent non-overlapping tile contains the first column of the vertical difference matrix;

h) repeating step f) iteratively for at least one adjacent non-overlapping tile in the horizontal difference matrix, using as input the stored values of the row immediately before said adjacent non-overlapping tile, or using the corresponding T elements of the horizontal input vector computed in step c) if there are no such values stored because said adjacent non-overlapping tile contains the first row of the horizontal difference matrix.

As previously mentioned, a major benefit of using this method is the significant memory footprint reduction, as it only requires storing the elements at the edges of the tiles without losing information.

In some cases, it may be interesting or necessary to extend the previously described method in order to perform a traceback of the computation. In this case, the following steps shall be performed after previous step h):

i) computing an alignment traceback of a first tile of dimension T x T f from a predefined starting position and from the stored values of the vertical difference matrix or from the vertical input vector computed in step c) if the first column is enclosed in said tile, and storing in the memory unit said alignment traceback and an ending position;

j) computing an alignment traceback of a first tile of dimension T x T f from a predefined starting position and from the stored values of the horizontal difference matrix or from the horizontal input vector computed in step c) if the first row is enclosed in said tile, and storing in the memory unit said alignment traceback and an ending position;

k) repeating step i) for computing the alignment traceback of at least one adjacent non-overlapping tile using as starting position the ending position computed in step i);

l) repeating step j) for computing the alignment traceback of at least one adjacent non-overlapping tile using as starting position the ending position computed in step j); and

m) combining the stored alignment tracebacks as computed in steps i) - l) to produce two modified or unmodified sequences with respect to the sequences received in step a) which maximize the degree of similarity between the sequences received in step a).

This allows for obtaining two or more final sequences in their optimal configuration (i.e., with the minimum number of mismatches, insertions and deletions) using a very reduced amount of memory and thus making the alignment method more efficient.

The steps e)-l) performed in said methods may be implemented through a set of instructions corresponding to an extension of an instruction set architecture (ISA), making them universal to implement in different CPUs or processors of other kind. Furthermore, in the case of applying the method to the analysis of genetic sequences, this, together with the specific hardware implementation described in the following, allows for its implementation in a complete genomics pipeline.

In another embodiment of the invention, the predetermined alignment algorithm used, and, thus, the one which is accelerated thanks to the use of the method, is based on bit-parallel techniques, and may be, in another embodiment of the invention the bit-parallel Myer's algorithm.

In another embodiment of the invention, the memory unit or memory units involved in the method are either scalar registers or vector registers. This is extremely related to the characteristics of another embodiment of the invention, in which the tile size T is half the value of the registers of the computation module's bit length. Notably, the tile size T has profound implications for the performance and efficiency of the method. Due to the fact that only the elements at the edge of each tile are stored in memory, the larger the value of T, the more efficient the method is, as less memory is required. Nevertheless, the sequences to be aligned are typically larger than the register's bit-length. In this case, two possibilities arise: either using scalar registers from a general-purpose processor selecting a value of T that maximizes the efficiency in these circumstances (i.e., half the value of the registers of the processor's bit length) or using vector registers allowing for larger values of T to be used. Generically, T = 32 when using the most common 64-bit scalar registers is the optimal value of T, although other possibilities may exist both for smaller values of T, and for larger values of T (using, for instance, bit-vector registers).

As previously mentioned, the present invention also proposes a computation module adapted to perform the previously described method that can be seamlessly integrated into any conventional CPU. This computation module comprises a so-called alignment computation module which, in turn, comprises:

- at least two architectural memory registers, each adapted to store a sequence of information; and
- a matrix of T × T compute cells adapted to perform a method according to any of claims 1-5 for accelerating an alignment algorithm to establish a correspondence between at least the two sequences of information stored in the architectural memory registers. Furthermore, each compute cell may be configured to compute one element of a T x T vertical difference matrix or of a T x T horizontal difference matrix.

This design can be implemented using a reduced number of gates, minimizing the propagation delay and the area footprint.

In another embodiment of the invention, the computation module further comprises at least two segmentation registers. As discusses above, large values of the tile size are optimal for the method's performance, but they require a careful segmented design to work at the processor's high clock frequencies. Concerning the computation module, a segmentation strategy is used introducing segmentation registers between the matrix antidiagonals, storing up to T elements in the worst case. This design can scale to arbitrarily large values of T and any number of stages, balancing the delay across them.

In another embodiment of the invention, the computation module is extended with a second module so that it is able to compute a traceback alignment. This second module or traceback module comprises:

three architectural registers adapted to store a traceback's start and/or end position, the 2T lower bits of an encoded alignment from a last traceback execution and a 2T higher bits of an encoded alignment from a last traceback execution, respectively; and

- a matrix structure of T x T core modules configured to perform a method for calculating the traceback of a vertical or horizontal difference matrix according to the method described above. Said matrix structure of core modules is further configured to use a path selector connected to the left, left-up, and up adjacent path selectors to determine the traceback of a tile and to store the final traceback position of said tile, the 2T lower bits of an encoded alignment from a last traceback execution and the 2T higher bits of an encoded alignment from a last traceback execution in the corresponding architectural registers.

Interestingly, in this way, the alignment path only traverses one path selector on each antidiagonal at most. This property is exploited by the invention to simplify the traceback module design, storing in two architectural registers the enabled path selector on each antidiagonal using 2-bits. The traceback module can also be implemented using a few gates to reduce the area and propagation delay.

The traceback module may comprise, in another embodiment of the invention, at least two segmentation registers. In this case, the delay of the traceback module must account for the alignment re-computation delay. Therefore, a segmentation strategy is also used in the traceback module design, which also involves using antidiagonals segmentation registers. First, the differences are computed and stored in all the segmentation registers. Then, the traceback of each segmented stage is computed. In practice, the traceback module needs to be segmented more times to achieve the same operating frequency. Furthermore, the traceback algorithmic phase is inherently sequential. Therefore, the traceback module design can be efficiently implemented as a multicycle model, reducing the design complexity.

In summary, the proposed method and corresponding computation module allow for accelerating alignment algorithms in order to aligning sequences of information in an efficient way in which the used memory is considerably smaller than in other methods of the art, achieving a fast, scalable, accurate, and efficient sequence analysis tool.

**BRIEF DESCRIPTION OF DRAWINGS**

FIG. 1 shows a schematic representation of the process of computing the alignment or best correspondence between two four-character genetic sequences according to a generic DP-based algorithm. Left panel shows the computation of the DP-matrix, central panel shows the traceback matrix and the traceback computed path, and right panel shows the final alignment between the two compared sequences.

FIG. 2 shows a schematic representation of the encoding of the DP-matrix showed in FIG. 1 using vertical and horizontal differences (left panel) as well as a more detailed description of a matrix element according to the Bit Parallel Myer's method.

FIG. 3 shows a schematic comparison between an element-by-element computation of a classical DP algorithm (left panel) a vector-by-vector computation of Bit Parallel Myer's method (central panel) and a computation according to the method of the present invention (right panel) as well as the memory storage requirements for each of them.

FIG. 4 shows a schematic representation of the implementation of the method of the invention to the computation of the alignment or best correspondence between two four-character genetic sequences, according to a preferred embodiment thereof. In this case, the method comprises the tile-by-tile computation of the vertical and horizontal difference matrices (steps 1-3 corresponding to the upper panels in the FIG.) and the tile-by-tile obtention of the alignment traceback (steps 4-6 corresponding to the lower panels in the FIG.).

FIG. 5 shows a schematic representation of the hardware design tailored to perform the method of the invention, according to a preferred embodiment thereof. The left panel shows matrix structure of the design with a tile size of T=3, whereas the right panel shows a single compute cell's structure.

FIG. 6 shows a schematic representation of the hardware design logic for the traceback computation of a 2x2 tile aligning two two-character genetic sequences.

FIG. 7 shows a schematic representation of the segmentation strategy both for the alignment computation (left panel) and for the traceback computation (right panel).

FIG. 8 shows a schematic representation of the tiles computed by three different implementations of the method of the invention using well-known alignment algorithms to perform said method.

FIG. 9A shows the comparison of the results (alignments per second) of the alignment of short sequences on a CPU enabled with the hardware and the method of the invention, for different well-known alignment algorithms.

FIG. 9B shows the comparison of the results (alignments per second) of the alignment of long sequences on a CPU enabled with the hardware and the method of the invention, for different well-known alignment algorithms.

FIG. 10A shows the comparison of the results (alignments per second) of the alignment of short sequences on a CPU enabled with the hardware and the method of the invention, for different well-known domain-specific accelerators.

FIG. 10B shows the comparison of the results (alignments per second) of the alignment of long sequences on a CPU enabled with the hardware and the method of the invention, for different well-known domain-specific accelerators.

### NUMERIC REFERENCES USED IN THE DRAWINGS

| (1) | Sequences of information |
|---|---|
| (2) | Dynamic programming (DP-) matrix |
| (3) | Matrix element |
| (4) | Traceback |
| (5) | Vertical difference matrix |
| (6) | Horizontal difference matrix |
| (7) | Tile |
| (8) | Tile size |
| (9) | Vertical input vector |
| (10) | Horizontal input vector |
| (11) | Computation module |
| (12) | Alignment computation (AC) module |
| (13) | Traceback (TB) module |
| (14) | Compute cell |
| (15) | Core module |
| (16) | Path selector |

### DETAILED DESCRIPTION OF THE INVENTION

A preferred embodiment of the invention, shown in FIG.s 1-10, will be now described for illustrative, but not limiting, purposes. This preferred embodiment aims to overcome the limitations present in the art, as exposed in the background section of the present application. These limitations relate to the fact that the alignment of sequences (1) of information (in different application domains) has typically relied upon dynamic programming (DP) techniques, that scale quadratically in both execution time and memory with the length of the sequences (1) involved.

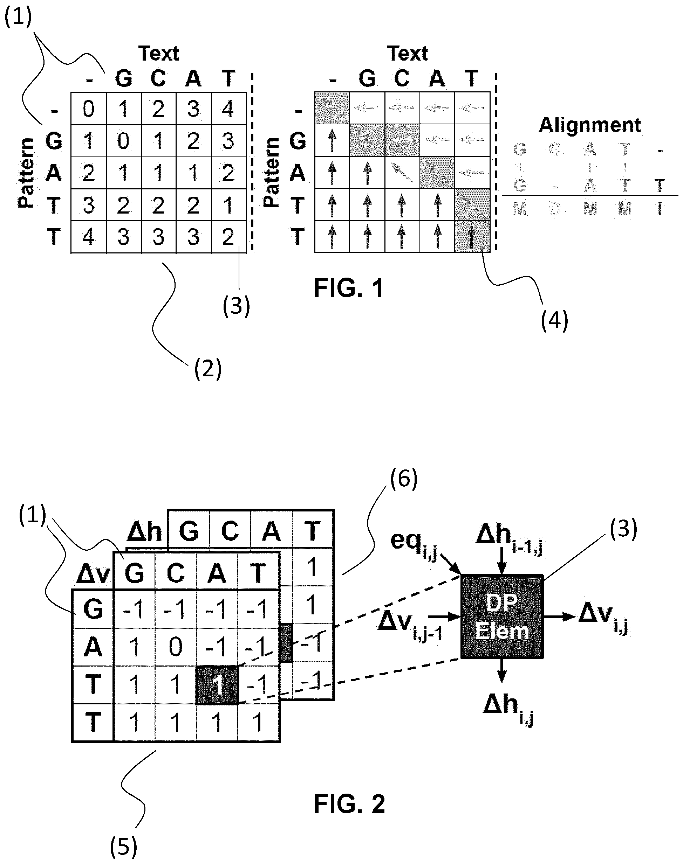

FIG. 1 shows an example of using one of these classical DP methods for the computation of the alignment between two four-character sequences (1): "CGAT" and "CATT". During the first phase (left panel), sequence alignment algorithms compute a DP-matrix (2) of $n \times m$ matrix elements (3) with $n$ and $m$ being the lengths of the sequences (1) to be aligned, in this case, $n = m = 4$ .

Then, for the traceback (4) phase (central panel), sequence (1) alignment algorithms trace the list of operations that lead to the optimum distance (bottom-right element of the matrix) back to the beginning (upper-left element); that is, the alignment between the sequences (1) (right panel).

More sophisticated methods, such as those exploiting bitwise operations to compute multiple elements (3) of the DP-matrix (2) in parallel, although better in performance and memory requirements, also suffer from severe limitations. For instance, the Bit Parallel Myer's benefits from the observation that differences between adjacent row and column elements are limited to {-1, 0, +1}. BPM exploits this property encoding vertical differences, $\Delta v_{i,j} = H_{i,j} - H_{i-1,j}$, and horizontal differences $\Delta h_{i,j} = H_{i,j} - H_{i,j-1}$, in a vertical difference matrix (5) and a horizontal difference matrix (6), respectively, with $H$ being the DP-matrix (2), requiring only ($2 \times 2$) bits per element of the original DP-matrix (2), irrespective of the alignment error. As an illustrative example, FIG. 2 shows the encoding of the DP-matrix (2) previously shown in FIG. 1 (and, thus, corresponding to the alignment of sequences (1) "CGAT" and "CATT") using vertical and horizontal differences (left panel) as well as a more detailed description of a vertical or horizontal difference matrix element (3) (right panel). This method, although improved with respect to the non-parallel versions of the DP-based algorithms, suffers as well from accuracy, scalability, efficiency, performance, and applicability limitations.

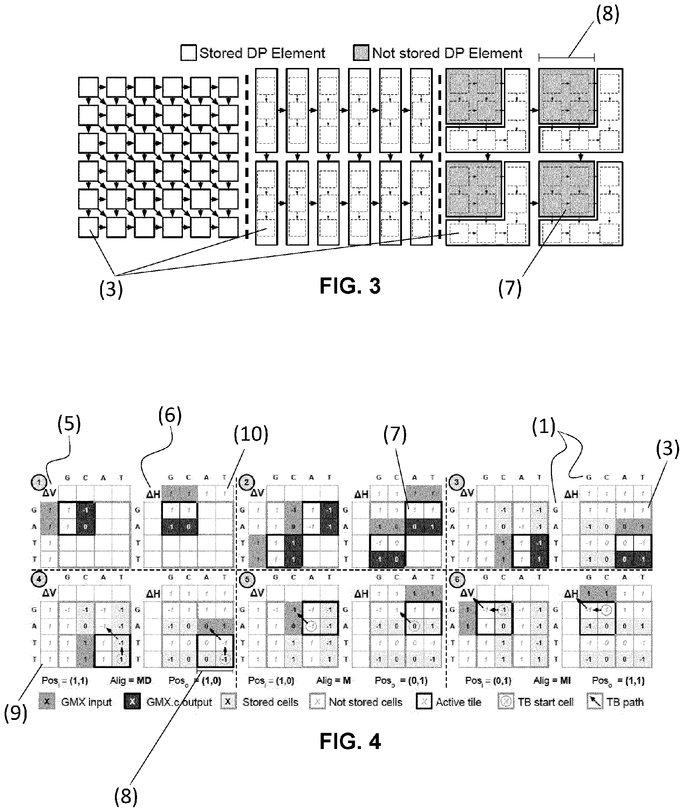

A preferred embodiment of the method of the invention overcomes these limitations by computing the vertical and horizontal difference matrices (5, 6) tile (7) by tile, as opposed to classical DP-based alignment algorithms that compute the DP-matrix element (3) by element, and to bit-parallel algorithms, like BPM, that compute multiple elements in a column leveraging CPU bitwise instructions. This increases the computational intensity, requiring only to store the elements at the edge of each tile. As a result, the computations are reduced quadratically with the tile size (8) while the memory requirements are significantly decreased. FIG. 3 shows a schematic comparison between classical DP-based alignment algorithms (left panel), bit-parallel algorithms (central panel) and the method of the invention (right panel).

Said preferred embodiment refers to a method in which, first, two sequences (1) are received and stored in a memory unit for comparison. From a predetermined state-of-the-art alignment algorithm, and, more specifically, from the scores associated to the comparison of the sequences (1) according to said alignment algorithm, two input vectors (a vertical input vector (9) and a horizontal input vector (10)) based on differential encoding are computed: one allocating the vertical differences and one allocating the horizontal differences. These two input vectors (9, 10), which define the input boundary conditions for the method, are then use to compute a first tile (7) of dimension T x T, with T being the tile size (8) corresponding to a predetermined value larger than 1, both for the vertical difference matrix (5) and for the horizontal difference matrix (6). Once this tile (7) is computed for each of these matrices (5, 6), only the column with the highest index within the tile (7) is stored in memory in the case of the vertical differences and only the row with the highest index within the tile (7) is stored in memory in the case of the horizontal differences. This tile computation is repeated for adjacent tiles (7), either using as input the last column (vertical differences) or the last row (horizontal differences) or the corresponding elements of the input vectors (9, 10) if the tile to be computed encloses the first column (vertical differences) or the first row (horizontal differences) of the matrix.

Once the whole matrices (5, 6) have been computed tile-wise, a traceback (4) procedure may be performed using a similar methodology if the overall problem requires it. For this matter, from a predefined starting position (typically the bottom-right corner of the matrices), the traceback (4) of a whole tile (7) of dimension T x T is computed and the ending position of such traceback (4) is stored in memory. This is done by recomputing all the elements (3) of the tile (7) taking as input those adjacent to it already stored in memory during the matrix computation phase described above. Once the traceback (4) is calculated from these values, the non-stored ones are discarded, avoiding using more memory than that strictly needed. The aligned sequences (1) corresponding to the traceback (4) of the tile are stored and the traceback (4) of a new, adjacent tile (7) is computed in the same way until the two entire sequences (1) have been aligned.

In a preferred embodiment of the invention, the previous method, that in following paragraphs may be dubbed GMX from *"Genomics Matrix eXtension"*, is implemented by providing a set of ISA extensions that features three custom instructions (gmx.v, gmx.h, and gmx.tb) and requires five architectural state registers of 2T-bits (gmx_s1, gmx_s2, gmx_pos, gmx_lo, and gmx_hi) used as memory units. For illustration and exemplification purposes of said embodiment, two sequences (1) of characters will be compared in order to be aligned, as encoded in the registers gmx_s1 and gmx_s2.

Schematically, the instructions and registers relate to each other in the following manner:

- gmx.v rd, rs1, rs2 . Given $\Delta v_i$ and $\Delta h_i$ (either input (9, 10) or stored vectors) stored in the general purpose registers rs1 and rs2, respectively, the gmx.v instruction computes a tile (7) of the vertical difference matrix (5) for the gmx_s1 and gmx_s2 corresponding, each of them, to one of the sequences (1) to be aligned. The resulting $\Delta v_0$ is stored in the register rd.
- gmx.h, rd, rs1, rs2. Given $\Delta v_i$ and $\Delta h_i$ (either input (9, 10) or stored vectors) stored in the general purpose registers rs1 and rs2, respectively, the gmx.v instruction computes a tile (7) of the horizontal difference matrix (6) for the gmx_s1 and gmx_s2 corresponding, each of them, to one of the sequences (1) to be aligned. The resulting $\Delta h_0$ is stored in the register rd.

- gmx.tb rs1, rs2. Given rs1= $\Delta v_i$, rs2= $\Delta h_i$ stored, and gmx_pos (traceback starting position), this instruction computes the alignment traceback (4) of a tile (7) for the gmx_s1 and gmx_s2. As a result, it produces gmx_lo and gmx_hi (4T-bit encoded alignment) and updates gmx_pos (traceback end position).

The architectural registers can be accessed using standard read-and-write instructions implemented in conventional ISAs (like the CSRRW instructions on RISC-V) and correspond to the following:

- gmx_s1: Stores one of the sequences (1) to be aligned.
- gmx_s2: Stores the other sequence (1) to be aligned.
- gmx_pos: Stores the traceback's start/end position.
- gmx_lo: Stores the 2T lower bits of the encoded alignment from the last traceback (4) execution.
- gmx_hi: Stores the 2T higher bits of the encoded alignment from the last traceback (4) execution.

It is to be noted that the size of the gmx_s1 and gmx_s2 architectural registers can be increased to allocate arbitrarily large alphabets (e.g., 1-byte ASCII or even 3-bytes CCCII), allowing the method of the invention to be extended to any alphabet. In addition, all the architectural registers of the method can be renamed to allow the implementation of the ISA extension in an out-of-order processor.

FIG. 4 shows an illustrative example of an implementation of the method of the invention according to the previously defined characteristics. In particular, the method's steps using a tile size (8) of 2 (T = 2) to align the sequences (1) "GCAT" and "GATT" computing the Vertical and horizontal difference matrices (5, 6) and the traceback (4) are shown. In each panel, the corresponding active tile (7) (the one which is being computed), input information, and stored information are shown for an easier understanding of the method of the invention.

First, the upper panels of FIG. 4 show the computation of the $\Delta v_{mat}$ and $\Delta h_{mat}$ matrices (5, 6) given the sequences (1) to be aligned, the vertical and horizontal input vectors (9, 10), and the tile size (8) T. The algorithm computes the complete vertical and horizontal difference matrices (5, 6), using T×T tiles (7), advancing column-wise. To compute each T×T tile (7), gmx_s1 and gmx_s2 must be set (using the csrw instruction), and the input differences vectors ($\Delta v_i$, $\Delta h_i$) (9, 10) must be loaded. After that, the gmx.v and gmx.h instructions are executed to generate the $\Delta v_0$ and $\Delta h_0$ vectors. These vectors are stored in $\Delta v_{mat}$ and $\Delta h_{mat}$ for future computations. It is to be noted that only the last $\Delta v$ column and the last $\Delta h$ row

of each tile (7) are needed to be stored, reducing by a factor of T the amount of memory footprint needed.

Second, the lower panels of FIG. 4 show the traceback (4) computation to retrieve the sequence alignment, given the sequences (1) to be aligned, the tile size (8), T, and the $\Delta v_{mat}$ and $\Delta h_{mat}$ computed on the first phase. The traceback (4) is computed for each tile (7) using the gmx.tb instruction and the corresponding gmx_s1, gmx_s2, and the input differences vectors ($\Delta v_i$, $\Delta h_i$) (9, 10), and this computation advances tile-wise from the last tile (7) (bottom-right corner) to the first tile (7) of the vertical and horizontal difference matrices (5, 6) (upper-left corner). The gmx.tb instruction starts the traceback (4) from the position set in gmx_pos, outputs the alignment encoded in gmx_hi and gmx_lo, and updates gmx_pos with the traceback's ending position (i.e., starting position of the next tile's traceback). This process is iterated until all the partial tracebacks (4) are computed.

The method of the invention is designed to be an instruction set extension for fast and accurate sequence analysis, focusing on genome sequence analysis and other applications as previously mentioned. Because these extensions must be located inside the processor pipeline, the method's implementation has specific hardware constraints:

- a small area footprint,
- a short execution latency (few clock cycles)
- the need to use the processor's general-purpose registers efficiently, and
- iv) achieve the same high frequency as the processor.

An embodiment of the invention refers to a computation module (11) configured to perform the method described above which, at the same time, meets all these hardware requirements, and allows for accelerating the computations of a tile (7) of the vertical and horizontal difference matrices (5, 6) while reducing the memory footprint. Said embodiment comprises two modules: one implementing the vertical and horizontal difference matrices (5, 6) computation (alignment computation (AC) module (12)) and another one implementing the traceback computation (traceback (TB) module (13)).

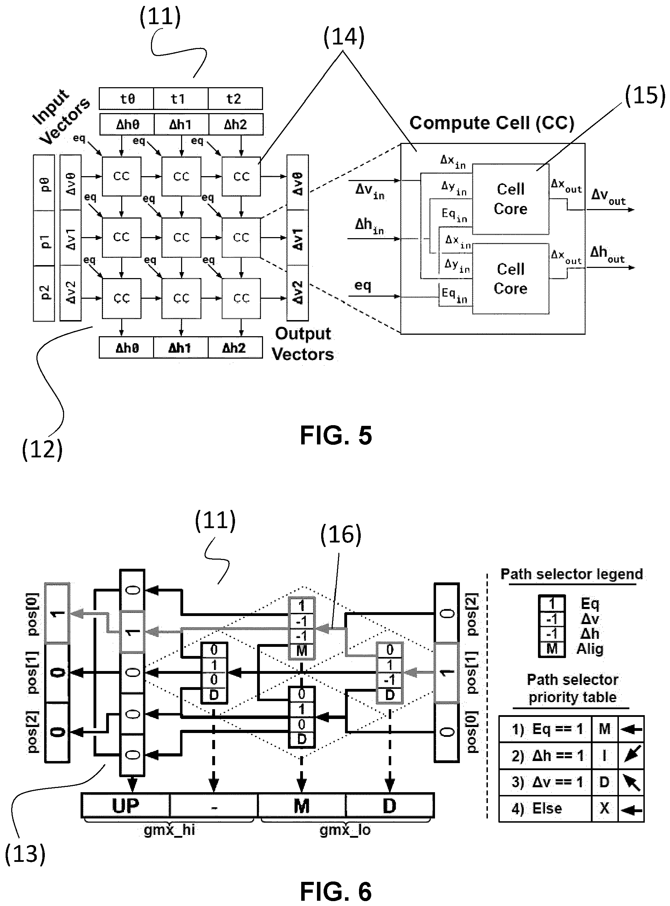

The AC module (12) consists of a matrix of T×T basic BPM compute cells (14) , $CC_{i,j}$, which inputs two T-size vectors of $\Delta v$ and $\Delta h$ (9, 10), gmx_s1, and gmx_s2, and outputs the resulting $\Delta v$ and $\Delta h$ vectors, executing gmx.v and gmx.h instructions, respectively. The general schematic representation of such compute cell (14) (in this case a 3x3 cell) is shown in the left panel of FIG. 5. In this AC module (12), the data flow starts from the top-left corner and the computation takes place towards the bottom-right corner.

As depicted in the right panel of FIG. 5, each compute cell (14) $CC_{i,j}$ computes a single DP-matrix element (3) (encoded as $\Delta v$ and $\Delta h$), using left $\Delta v_{i,j-1}$, upper $\Delta h_{i-1,j}$, and the equality bit $eq_{i,j}$. The equality bit $eq_{i,j}$ is generated comparing the corresponding two characters from gmx_s1 and gmx_s2. It is to be noted that all difference values are encoded in 2-bits. Internally, each compute cell (14) implements two cell core modules (15) to compute $\Delta v$ and $\Delta h$. Exploiting the symmetries of the BPM technique, both core modules (15) implement the same operation.

After the vertical and horizontal difference matrices (5, 6) computation, the presented extensions allow to traceback (4) the alignment operations of each tile by using the TB module (13). In order to do that, a matrix-like structure of core modules (15) as the one exemplified in FIG. 6 is employed. This module's (13) inputs are $\Delta v$, $\Delta h$, gmx_s2, gmx_s1, and the architectural register gmx_pos. Unlike in the AC module (12), in the TB module (13) the data flow starts from the bottom-right corner towards the top-left corner. Moreover, because the traceback (4) can start at any element of the bottom and right of the tile (7), the register gmx_pos encodes the starting position of the traceback (4).

Each TB core module (15) uses a path selector (16) to discriminate which adjacent element (3) belongs to the alignment path using the following encoding: match/mismatch=↖, insertion=←, and deletion=↑. Each path selector (16) is connected to the three adjacent path selectors (16) (left, left-up, and up) and enables one depending on $\Delta v$, $\Delta h$, and $eq_{i,j}$. This alignment path is propagated until it reaches a left or top edge. The position of the last path selector (16) is stored in gmx_pos for the next tile traceback (4) computation.

Moreover, the sequence of path selectors (16) enabled (i.e., the alignment path) is stored into the gmx_hi and gmx_lo registers.

Notably, the tile size (8) T has profound implications for the performance and efficiency of the method of the invention. The embodiments corresponding to the computation module (11) or hardware implementation disclosed herein are designed to exploit the available general-purpose registers efficiently. For that, an optimal selection of the tile size (8) T allows for utilizing the whole register's length. Thus, although the larger the value of T, the more efficient the method is, as less memory is required, such value of T must be selected according to the processor's and memory's specifications. Nevertheless, large values of T require a careful segmented design to work at the processor's high clock frequencies.

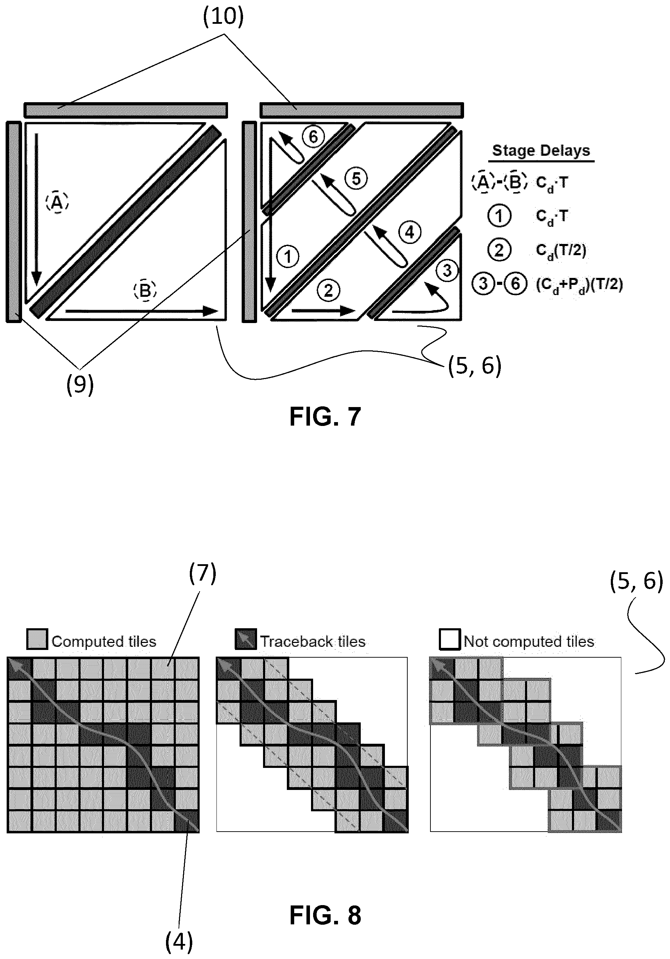

Concerning the AC module (12), the maximum delay paths start on the top-left compute cell (14) and finish at the bottom-right compute cell (14), traversing 2T - 1 compute cells (14). Being $C_d$ the delay of a AC module (12) compute cell (14), the critical path of this AC module (12) is $(2T-1) \cdot C_d$. For T = 32, the critical path is then 63 $\cdot C_d$. Even for small $C_d$, a single-cycle implementation of the AC module (12) will not reach the high clock frequencies delivered by modern CPUs. A segmentation strategy is therefore used in the AC module (12) design. The segmentation strategy schematics of this AC module (12) are shown in the left panel of FIG. 7. This procedure introduces segmentation registers between the matrix antidiagonals, storing up to T elements in the worst case. This design can scale to arbitrarily large values of T and any number of stages, balancing the delay across them. For instance, a two-cycle segmented design for T = 32 renders two stages of delay 32 $\cdot C_d$.

Regarding the TB module (13), the delay of the traceback (4) goes from the bottom-right to the top-left corner, going through 2T-1 path selectors (16), each one introducing $P_d$ delay. However, the overall delay of the TB module (13) must account for the AC module (12) re-computation delay. That is, a $((2T - 1)( C_d + P_d))$ total traceback delay. A segmentation strategy is therefore used in the TB module (13) design. The segmentation strategy schematics of this TB module (13) are shown in the right panel of FIG. 7. The TB module (13) segmentation strategy also involves using antidiagonals segmentation registers. First, the differences are computed and stored in all the segmentation registers. Then, the traceback (4) of each segmented stage is computed. In practice, the TB module (13) needs to be segmented more times to achieve the same operating frequency as the AC module (12). Furthermore, the traceback algorithmic phase is inherently sequential. Therefore, the TB module design can be efficiently implemented as a multicycle model, reducing the design complexity.

In order to demonstrate the performance of the method of the invention and its corresponding computation module, in the following the results of the acceleration of three different algorithms by using said method and computation module are presented.

A 64-bit Linux capable processor fabricated in GlobalFoundries 22nm FDSOI technology node has been used and a tile size (8) of T=32 has been selected in order to maximize 64-bit registers usage. Using this configuration, using the method and computation module (11) (hardware implementation) of the present invention, 1024 elements can be computed per instruction. To achieve a 1 GHz working frequency, the AC and TB modules (12, 13) are segmented to obtain 2 and 6-cycle operation latency, respectively. Furthermore, an edge RISC-V processor (RV64G instruction set) has been used, featuring a single-core, 7-stage, in-order, single-issue pipeline. The memory hierarchy implemented in the System on a Chip (SoC) features non-blocking L1 and L2 data caches having sizes of 32KB and 512KB, respectively. Performance results have been obtained emulating the entire SoC in a Xilinx Alveo U280 FPGA.

The SoC design, including the implementation of the invention, has been synthesized in the GlobalFoundries 22nm FD-SOI technology node. The physical design targets post-routing clock timing 1GHz. A Cadence Genus tool v19.11 for the logical synthesis and a Cadence Innovus tool v19.11 for the place-and route have been used. To extract the design's power consumption accurately, utilization information reported from gate-level simulations of the entire SoC running sequence alignment benchmarks has been performed.

For the evaluation, 5 short-sequence datasets and 22 long-sequence datasets have been generated. The long-sequence datasets have different sequence lengths (from 1K bases to 10K bases in 1K base steps) and show error rates between 10% and 15%. The short-sequences datasets contain sequences (1) of length 100bps, 150bps, 200bps, 250bps, and 300bps, with an error rate of 5%. Additionally, a 1 Mbps long sequences dataset at 15% error rate has also been generated.

To evaluate the method of the invention's performance improvements, three widely used sequence alignment algorithms have been selected, ported, and executed into the RISC-V in-order processor. A classical dynamic programming implementation of the sequence alignment has been firstly evaluated as the baseline. Then, the original BPM, the Edlib algorithm, and the open-source CPU-based GenASM implementation have been tested with and without their corresponding enhanced version by using the method and computation module (11) of the invention.

The three different approaches can be seen schematically in FIG. 8, in which, for each of the algorithms, the number of tiles (7) computed highlighting the tiles traversed during the traceback (4) are shown.

1) BPM (left panel of FIG.8): The method of the invention can be used to accelerate the classical BPM to compute the complete DP-matrix (2) tile-wise. While the classical BPM requires 17 bitwise operations to compute a column of 64 elements (3), its accelerated version due to the use of the method of the invention only needs 2 instructions to compute a $(T \times T)$-elements tile (7). Moreover, BPM is limited to computing the traceback (4) elementwise, while its accelerated version due to the use of the method of the invention computes the traceback (4) of an entire tile (7) per instruction, reporting the same results. Regarding memory usage, BPM requires storing all the differences $(4 \times n \times m$ bits) to compute the traceback (4). In contrast, its accelerated version due to the use of the method of the invention only stores the differences at the tile edges, consuming a total memory of $\frac{4 \times n \times m}{T}$, requiring T times less memory than the classical BPM.

2) Edlib: The Banded Algorithm Edlib is a widely used sequence alignment algorithm that implements the BPM augmented with heuristics. Edlib's algorithm involves computing a band of elements (3) along the main diagonal of the DP-matrix (2) (central panel of FIG. 8). The band size is dynamically determined to guarantee the accuracy of the alignment. As opposed to BPM, Edlib only computes the $m \times B$ elements (3), where $B$ is the band size. In the same spirit, its accelerated version due to the use of the method of the invention applies the same band heuristic to reduce computations to $\frac{m \times B}{T^2}$ tiles (7), while reducing the memory footprint from $4 \times B \times m$ to $\frac{4 \times B \times m}{T}$.

3) GenASM: To be able to scale to long sequence alignments, GenASM proposes a divide-and-conquer approach. Instead of computing the entire sequence alignment, sequences (1) are divided into smaller overlapping windows of size W×W. Starting from the last window (placed at the bottom-right of the DP-matrix (2)), the algorithm computes the partial tracebacks (4) allowing an overlap of size 0 between succeeding windows. To obtain the final alignment, the algorithm joins all the partial tracebacks (4) from the overlapping windows. GenASM's algorithm can be conveniently accelerated using the method of the invention by mapping overlapping windows to tiles (7) (right panel of FIG. 8). As a result, the memory footprint of GenASM is reduced from $(W^3)$ bits to ( $4 \times \frac{W^2}{T}$ ) (i.e., a $\frac{TW}{4}$ reduction in memory).

The first results concern chip area, frequency, and power analyses. In this sense, the silicon area occupied by the computation module (11) of the present invention only represents 1.7% of the entire SoC (0.0216 $mm^2$, of which 0.0087 $mm_2$ correspond to the AC module (12) and 0.0108 $mm^2$ to the TB module (13)). Moreover, each of the AC and TB modules (12, 13) consumes an area similar to that of a 2-cycle 64-bit integer multiplier. Regarding power consumption, the AC and TB modules (12, 13) only increase by 8.47 mW the SoC power consumption which represents 2.1% of the total power consumption.

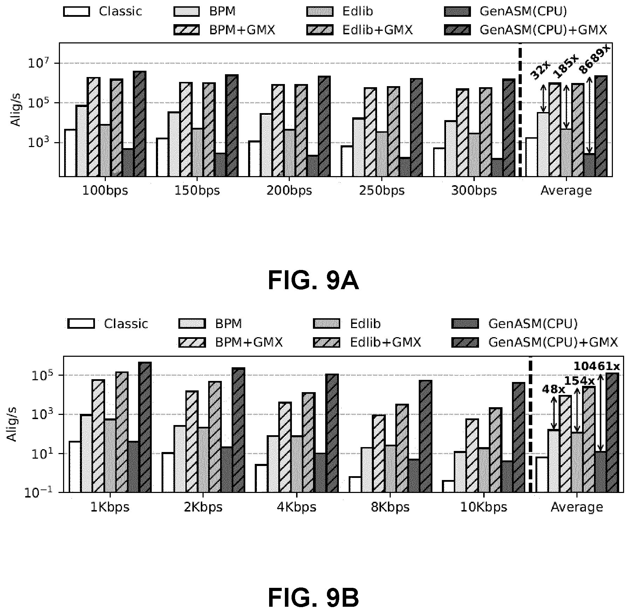

The second results correspond to the performance improvements obtained by using the present invention upon the three previously mentioned well-known algorithms implemented on a simple in-order single-issue pipeline. FIG. 9A and FIG. 9B show the throughput (alignments per second) obtained by the original and by the accelerated versions of BPM, Edlib, and CPU-based GenASM algorithms, aligning short and long sequences (1). The classic DP-based algorithm is also included as a baseline.

In FIG. 9A it is evident that, regarding short-sequence alignment, the use of the method and computation module (11) of the invention achieves 32 times more throughput than the original BPM. In the case of Edlib, the acceleration achieves a 185 times larger throughput improvement. Similarly, in the case of GenASM, the use of the invention improves the throughput by a factor of 8689.

FIG. 9B shows the equivalent results for long-sequence alignment, where a 48 times larger throughput improvement in BPM, a 154 times larger throughput improvement in Edlib, and a 10461 times larger throughput improvement in GenASM are achieved by using the method and computation module (11) of the invention.

To demonstrate the invention's scalability, 1Mbpslong sequences (1) with 15% of error have been aligned using the accelerated versions of Edlib and GenASM by implementing the disclosures of the invention. Compared with their non-accelerated versions, an improvement of 1.58 times more throughputs has been achieved without the need to modify any design parameter or increase internal SRAM memories.

Regarding accuracy, the accelerated versions of BPM and Edlib always reach 100% accuracy as both underlying algorithms are exact, and the use of the method and computation module (11) of the invention does not sacrifice accuracy. For GenASM based implementations, its accelerated version using W = 96 and O = 32 still achieves a 100% accuracy, whereas, using smaller window sizes, like the one used in the original GenASM study (W = 64 and O = 24), average accuracy drops to 98% when aligning short sequences (1), and down to 89% when aligning long sequences (1).

In the same spirit, and in order to motivate the applicability of the invention, its results have been compared to those of the popular gap-affine model error. To that end, the alignment distance deviation between the edit distance and the gap-affine scoring has been evaluated. The performed experiments show that the resulting edit alignments introduce a 0.94% score deviation for short sequence alignments and a 2.1% for long sequence alignments. Due to the minor score deviation obtained, it can be concluded that the invention can also be of interest to applications that use other error models.

For the comparison with domain-specific accelerators, state-of-the-art hardware accelerators GACT (from Darwin) and GenASM have been selected. For a fair comparison, a single core equipped with a single unit of the computation module (11) of the invention, one GenASM's vault (i.e., processing unit), and one Darwin's GACT 64-element array have been compared so that the three scenarios did not suffer from having limited memory bandwidth. The first one benefits from the cache hierarchy inside the SoC, while the other two use internal SRAMs.

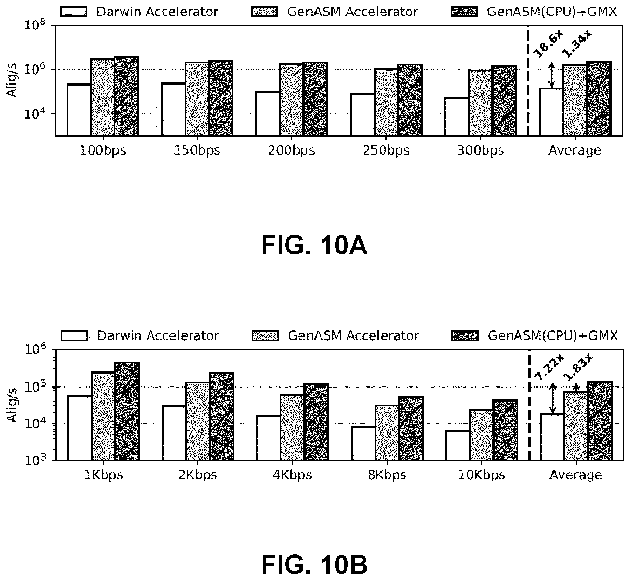

The results of the comparison are presented in FIG. 10A and FIG. 10B, showing the obtained throughput (alignments per second) aligning short and long sequences (1), respectively, by the three hardware accelerators.

FIG. 10A shows that, on average, the method and computation module (11) of the invention outperforms GenASM by a factor of 1.34 and GACT by a factor of 18.6. Comparing the silicon area, the computation module (11) of the invention requires 15.46 times less area than GenASM and 26.29 times less than GACT. Thus, using the invention provides a 13.2 and 310.7times

better throughput per mm$^2$ compared to GenASM and GACT, respectively. Regarding power efficiency, the method and computation module (11) of the invention provide 16.20 times and 655.74 times better throughput per watt than GenASM and GACT, respectively.

For long sequence alignment, (FIG. 10B), the method and computation module (11) of the invention provide 1.8 times more throughput than GenASM and 7.1 times more throughput than Darwin's GACT on average. Moreover, they provide 28.26 times and 189.79 times better throughput per silicon mm$^2$ compared to GenASM and GACT, respectively, when aligning long sequences (1). Regarding the power efficiency, they provide 21.84 times and 239.64 times better throughput per watt than GenASM and GACT, respectively.

These results demonstrate that the method of the invention enables the acceleration of sequence alignment by computing the DP-matrix (2) tile (7) by tile (7) instead of element (3) by element (3). Moreover, the proposed corresponding implementation in a tailored designed computation module (11) supposes an area- and energy-efficient hardware implementation that can be integrated into any CPU. After integrating the disclosures of the invention in an in-order RISC-V edge processor, it is shown that accelerated algorithms outperform state-of-the-art software tools and domain-specific accelerators both in performance and efficiency without degrading accuracy and scalability.

## Claims

1. Computer-implemented method for accelerating an alignment algorithm to establish a correspondence between at least two sequences (1) of information, the method comprising the following steps:

   a) receiving, in a computation module, at least two sequences (1) of information;
   b) storing said sequences in at least one memory unit of a computation module (11);
   c) computing, with the computation module (11), a vertical input vector (9) and a horizontal input vector (10) based on differential encodings of scores associated to the comparison of the sequences (1) according to the alignment algorithm to be accelerated;

   and **characterized in that** the method further comprises the following steps:

   d) setting a value corresponding to a matrix tile size (8), T, being said value an integer larger than 1;
   e) computing, from the first T elements of the vertical input vector (9) determined in step c), a vertical difference matrix (5) of a first tile (7) of dimension T x T according to the alignment algorithm, and storing in the memory unit the values of the column with the highest column index within the tile (7);

f) computing, from the first T elements of the horizontal input vector (10) determined in step c), a horizontal difference matrix (6) of a first tile (7) of dimension T x T according to the alignment algorithm, and storing in the memory unit the values of the row with the highest row index within the tile (7);

g) repeating step e) iteratively for at least one adjacent non-overlapping tile (7) in the vertical difference matrix (5), using as input the stored values of the column immediately before said adjacent non-overlapping tile (7), or using the corresponding T elements of the vertical input vector (9) computed in step c) if there are no such values stored because said adjacent non-overlapping tile (7) contains the first column of the vertical difference matrix (5);

h) repeating step f) iteratively for at least one adjacent non-overlapping tile (7) in the horizontal difference matrix (6), using as input the stored values of the row immediately before said adjacent non-overlapping tile (7), or using the corresponding T elements of the horizontal input vector (10) computed in step c) if there are no such values stored because said adjacent non-overlapping tile (7) contains the first row of the horizontal difference matrix (6).

2. Method according to the preceding claim, where the alignment algorithm to be accelerated is based, at least in part, on bit-parallel techniques.

3. Method according to the preceding claim, where the alignment algorithm to be accelerated comprises the bit-parallel Myer's algorithm.

4. Method according to any of the preceding claims, where the at least one memory unit comprises either a scalar register or a vector register.

5. Method according to the preceding claim, where the value of the tile size (8) T is half the value of the register's bit length.

6. Method according to any of the preceding claims, further comprising the following steps:

i) computing an alignment traceback (4) of a first tile (7) of dimension T x T f from a predefined starting position and from the stored values of the vertical difference matrix (5) or from the vertical input vector (9) computed in step c) if the first column is enclosed in said tile (7), and storing in the memory unit said alignment traceback (4) and an ending position;

j) computing an alignment traceback (4) of a first tile (7) of dimension T x T f from a predefined starting position and from the stored values of the horizontal difference matrix (6) or from the horizontal input vector (10) computed in step c) if the first row is enclosed in said tile (7), and storing in the memory unit said alignment traceback (4) and an ending position;

k) repeating step i) for computing the alignment traceback (4) of at least one adjacent non-overlapping tile (7) using as starting position the ending position computed in step i);

l) repeating step j) for computing the alignment traceback (4) of at least one adjacent non-overlapping tile (7) using as starting position the ending position computed in step j); and

m) combining the stored alignment tracebacks (4) as computed in steps i) - l) to produce two modified or unmodified sequences with respect to the sequences (1) received in step a) which maximize the degree of similarity between the sequences (1) received in step a).

7. Computation module (11) comprising an alignment computation module (12) comprising:

- at least two architectural memory registers, each adapted to store a sequence (1) of information; and
- a matrix of T x T compute cells (14) adapted to perform a method according to any of claims 1-5 for accelerating an alignment algorithm to establish a correspondence between at least the two sequences (1) of information stored in the architectural memory registers.

8. Computation module (11) according to the preceding claim, where each compute cell (14) is configured to compute one element (3) of a T x T vertical difference matrix tile (7) or of a T x T horizontal difference matrix tile (7).

9. Computation module (11) according to any of claims 7-8, further comprising at least two segmentation registers.

10. Computation module (11) according to any of claims 7-9, further comprising a traceback module (13) comprising:

- three architectural registers adapted to store a traceback's start and/or end position, the 2T lower bits of an encoded alignment from a last traceback execution and a 2T higher bits of an encoded alignment from a last traceback execution, respectively; and
- a matrix structure of T x T core modules (15) configured to perform a method according to claim 6.

11. Computation module (11) according to the preceding claim, where the matrix structure of core modules (15) is further configured to use a path selector (16) connected to the left, left-up, and up adjacent path selectors (16) to determine the traceback (4) of a tile (7) and to store the final traceback position of said tile (7), the 2T lower bits of an encoded alignment from a last traceback execution and the 2T higher bits of an encoded alignment from a last traceback execution in the corresponding architectural registers.

12. Computation module (11) according to the preceding claim, wherein the traceback module (13) further comprises at least two segmentation registers.

13. Computation module (11) according to any of claims 7-12, where said computation module (11) is further adapted to be connected or integrated to/in a general-purpose processor.

14. Use of a method according to any of claims 1-6 or of a computation module (11) according to any of claims 7-13 for establishing a correspondence between at least two:

    - genetic sequences,
    - time-series data sequences,
    - pattern matching sequences.

EP 4 394 777 A1

FIG. 1

FIG. 2

15

FIG. 3

FIG. 4

**FIG. 5**

**FIG. 6**

(10)

**Stage Delays**

$(A) - (B)$   $C_d \cdot T$

①   $C_d \cdot T$

②   $C_d (T/2)$

③ - ⑥   $(C_d + P_d)(T/2)$

(9)

(5, 6)

## FIG. 7

(7)

(5, 6)

☐ Computed tiles    ■ Traceback tiles    ☐ Not computed tiles

(4)

## FIG. 8

**FIG. 9A**

**FIG. 9B**

**FIG. 10A**

**FIG. 10B**

Europäisches
Patentamt

European
Patent Office

Office européen
des brevets

# EUROPEAN SEARCH REPORT

**Application Number**

EP 22 38 3313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/166218 A1 (FARIVAR REZA [US] ET AL) 27 June 2013 (2013-06-27) | 1-6 | INV.<br>G16B30/10<br>G16B50/30 |
| Y | * paragraphs [0018], [0019], [0021], [0023], [0027], [0029], [0041]; figures 1,2,3,5 * | 7-14 | |
| A | GENE MYERS: "A fast bit-vector algorithm for approximate string matching based on dynamic programming", JOURNAL OF THE ASSOCIATION FOR COMPUTING MACHINERY, ACM, NEW YORK, NY, US, vol. 46, no. 3, 1 May 1999 (1999-05-01), pages 395-415, XP058340666, ISSN: 0004-5411, DOI: 10.1145/316542.316550 * the whole document * | 1-14 | |
| Y | GUDUR VENKATESHWARLU YELLASWAMY ET AL: "Hardware-Algorithm Codesign for Fast and Energy Efficient Approximate String Matching on FPGA for Computational Biology", 2022 44TH ANNUAL INTERNATIONAL CONFERENCE OF THE IEEE ENGINEERING IN MEDICINE & BIOLOGY SOCIETY (EMBC), IEEE, 11 July 2022 (2022-07-11), pages 87-90, XP034183140, DOI: 10.1109/EMBC48229.2022.9870924 [retrieved on 2022-09-08] * Abstract * | 7-14 | **TECHNICAL FIELDS SEARCHED (IPC)**<br><br>G16B |

-/--

The present search report has been drawn up for all claims

| Place of search | Date of completion of the search | Examiner |
|---|---|---|
| Munich | 22 April 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Europäisches
Patentamt
European
Patent Office
Office européen
des brevets

**Application Number**

EP 22 38 3313

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIMURA KOUICHI ET AL: "A BIT-PARALLEL DYNAMIC PROGRAMMING ALGORITHM SUITABLE FOR DNA SEQUENCE ALIGNMENT", JOURNAL OF BIOINFORMATICS AND COMPUTATIONAL BIOLOGY, vol. 10, no. 04, 23 August 2012 (2012-08-23), page 1250002, XP093052781, GB ISSN: 0219-7200, DOI: 10.1142/S0219720012500023 Retrieved from the Internet: URL:http://dx.doi.org/10.1142/S02197200125 00023> * the whole document * | 1-14 | |
| A | SOSIC MARTIN ET AL: "Edlib: a C/C?++ library for fast, exact sequence alignment using edit distance", BIOINFORMATICS, vol. 33, no. 9, 1 May 2017 (2017-05-01), pages 1394-1395, XP093053478, GB ISSN: 1367-4803, DOI: 10.1093/bioinformatics/btw753 Retrieved from the Internet: URL:https://academic.oup.com/bioinformatic s/article-pdf/33/9/1394/49038512/bioinform atics_33_9_1394.pdf> * the whole document * | 1-14 | |

TECHNICAL FIELDS
SEARCHED      (IPC)

-/--

The present search report has been drawn up for all claims

| Place of search | Date of completion of the search | Examiner |
|---|---|---|
| Munich | 22 April 2024 | Mühlbauer, Max |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

&  : member of the same patent family, corresponding
     document

EPO FORM 1503 03.82 (P04C01)

# EUROPEAN SEARCH REPORT

**Application Number**

EP 22 38 3313

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WEIHONG XU ET AL: "RAPIDx: High-performance ReRAM Processing in-Memory Accelerator for Sequence Alignment", ARXIV.ORG, CORNELL UNIVERSITY LIBRARY, 201 OLIN LIBRARY CORNELL UNIVERSITY ITHACA, NY 14853, 7 December 2022 (2022-12-07), XP091388113, * the whole document * | 1-14 | |
| A | DAMLA SENOL CALI ET AL: "GenASM: A High-Performance, Low-Power Approximate String Matching Acceleration Framework for Genome Sequence Analysis", ARXIV.ORG, CORNELL UNIVERSITY LIBRARY, 201 OLIN LIBRARY CORNELL UNIVERSITY ITHACA, NY 14853, 16 September 2020 (2020-09-16), XP081764413, * the whole document * | 1-14 | |
| A | YONGCHAO LIU ET AL: "GSWABE: faster GPU-accelerated sequence alignment with optimal alignment retrieval for short DNA sequences", CONCURRENCY AND COMPUTATION: PRACTICE AND EXPERIENCE, WILEY, LONDON, GB, vol. 27, no. 4, 9 September 2014 (2014-09-09), pages 958-972, XP072310467, ISSN: 1532-0626, DOI: 10.1002/CPE.3371 * the whole document * | 1-14 | **TECHNICAL FIELDS SEARCHED (IPC)** |

The present search report has been drawn up for all claims

| Place of search | Date of completion of the search | Examiner |
|---|---|---|
| Munich | 22 April 2024 | Mühlbauer, Max |

EPO FORM 1503 03.82 (P04C01)

**EP 4 394 777 A1**

## ANNEX TO THE EUROPEAN SEARCH REPORT
## ON EUROPEAN PATENT APPLICATION NO.

EP 22 38 3313

22-04-2024

| Patent document cited in search report | Publication date | Patent family member(s) | Publication date |
|---|---|---|---|
| US 2013166218 A1 | 27-06-2013 | NONE | |

**EP 4 394 777 A1**

REFERENCES CITED IN THE DESCRIPTION

**Patent documents cited in the description**

- US 20210048992 A1